# FASCICULE DE BREVET EUROPEEN

(11) **EP 0 968 703 B1**
(45) Date de publication et mention de la délivrance du brevet: **24.03.2004**
(21) Numéro de dépôt: 99401282.1
(22) Date de dépôt: 28.05.1999
(51) Int. Cl.: A61K 7/00

(54) **Composition cosmétique ou pharmaceutique se présentant sous forme de solide et pouvant être déformable**
Kosmetische oder pharmazeutische Zusammensetzung in Form eines Feststoffs, welche verformt werden kann
Cosmetic or pharmaceutical composition under solid form and being deformable

(30) Priorité: 15.06.1998 FR 9807517
(43) Date de publication de la demande: 05.01.2000
(73) Titulaire: L'OREAL, 75008 Paris (FR)
(72) Inventeur: Gabin, Gérard, 75009 Paris (FR)
(74) Mandataire: Dodin, Catherine

(56) Documents cités:
- EP-A- 0 745 379
- WO-A-97/17055
- GB-A- 2 291 429
- US-A- 4 202 878
- US-A- 4 994 264
- US-A- 5 034 216
- US-A- 5 385 729

## Description

La présente invention a trait à une composition cosmétique ou pharmaceutique, se présentant sous forme d'un solide pouvant être déformable.

Certaines compositions cosmétiques se présentent sous forme de liquides ou de crèmes plus ou moins visqueux. Plus les compositions sont liquides, plus il est difficile de les prendre dans les mains; en effet, ces compositions ont tendance à s'échapper entre les doigts. Par ailleurs, elles nécessitent également l'utilisation de conditionnements étanches pour éviter les fuites de produit.
De façon simplifiée, une composition capillaire comprend généralement un ou plusieurs actifs tels que des détergents, des colorants, des actifs conditionneurs et/ou des actifs de permanente, dans un support ou milieu cosmétiquement acceptable contenant une grande quantité d'eau et le plus souvent des agents tensioactifs. Après chaque traitement spécifique des cheveux (shampooing, permanente, coloration, etc.), il est généralement nécessaire de rincer les cheveux afin de ne garder sur ceux-ci que les actifs traitants et éliminer le support, notamment les agents tensioactifs. Malheureusement, un grand nombre de compositions capillaires présentent l'inconvénient d'être difficiles à rincer et/ou de laisser des traces de produit sur les cheveux, leur conférant notamment un aspect collant, ciré, poisseux.
Il a été proposé, par la demande EP0692248, une composition capillaire nouvelle, permettant de remédier aux inconvénients mentionnés ci-dessus. En particulier cette composition se rince de façon remarquable et présente une texture tout à fait inhabituelle. En outre, elle est simple à appliquer. Cette composition comprend, dans un milieu cosmétiquement acceptable, un agent structurant insoluble dans ce milieu et formé de particules solides, conférant à la composition un aspect de solide déformable dans lequel le milieu est emprisonné, cet agent étant apte à s'éliminer au moyen d'un diluant.
Il a également été proposé, par la demande EP0692240, une composition destinée au traitement ou au nettoyage de la peau, qui contient des actifs, associés ou non à des détergents, et qui se rince de façon remarquable, tout en présentant une texture tout à fait inhabituelle, simple à appliquer.

On connaît également par US5034216, une composition cosmétique comprenant un copolymère éthylène/acrylate en une quantité de 1-6% en poids, associé à de la silice en une quantité de 1-30% en poids.
On connaît par US4994264, une composition cosmétique comprenant une poudre lubrifiante, des gommes hydrocolloidales, notamment des dérivés de cellulose, et des agents de liaison tel que l'amidon de maïs.
Le document US5385729 est relatif à une composition sous forme de gel comprenant un polymère réticulé épaississant et un agent abrasif.
Le document EP745379 est relatif à une composition solide expansée amidonnée comprenant un amidon naturel, la farine de blé.

WO97/17055 est relatif à un gel rigide comprenant des agents gélifiants hydrosolubles, dont notamment la gomme (ou farine) de Caroube ou un Carbomer.
GB2291429 décrit une composition comprenant 35-80% en poids de poudre, 10-25% en poids de tensioactif et 5-28 en poids de base anhydre.

Toutefois, ces compositions pour le traitement ou le nettoyage de la peau ou des cheveux présentent quelques difficultés à se déliter au contact d'un liquide, notamment au contact d'eau, froide ou même chaude, ce qui ne facilite pas leur emploi. Elles nécessitent par ailleurs l'utilisation d'assez grandes quantités d'agent structurant, ce qui peut nuire à leurs propriétés cosmétiques. Enfin, ces compositions ont par ailleurs tendance à se dessécher au cours de leur vieillissement.

La présente invention a pour but de résoudre ce problème et de proposer une composition qui se présente sous forme de solide pouvant être déformable, et qui soit susceptible de se déliter aisément au contact d'un liquide de préférence aqueux.

Par 'délitage' au sens de la présente invention, on entend un délitage à l'aide d'un liquide et non pas un délitage au toucher comme cela est le cas pour certaines compositions de maquillage de type fards à paupières qui peuvent être prélevées au doigt ou à l'aide d'un pinceau.
Ce délitage à l'aide d'un liquide correspond en fait à une déstructuration du solide, avec une solubilisation ou une mise en dispersion des particules dans le liquide.

La présente invention a donc pour objet une composition cosmétique ou pharmaceutique se présentant sous forme de solide, pouvant être déformable, et comprenant, dans un milieu cosmétiquement ou pharmaceutiquement acceptable, au moins un agent structurant, caractérisée par le fait qu'elle comprend en outre au moins un agent absorbant organique.
L'invention a également pour objet l'utilisation d'une telle composition pour le traitement cosmétique de la peau du visage et du corps, des cheveux, du cuir chevelu ou des muqueuses.

La composition ainsi obtenue présente l'avantage de se disperser rapidement et facilement dans un liquide, ce qui permet son utilisation aisée.
De plus, la composition se présentant de préférence sous forme d'un solide déformable, elle ne s'écoule pas entre les doigts.
D'autre part, elle présente un grand confort à l'application; par exemple, on ne constate aucune coulure de la composition risquant d'irriter notamment le visage et les yeux. Cette absence de coulure est très appréciée dans le cas des permanentes et des colorations, ainsi que pour les shampooings destinés aux enfants.
La composition selon l'invention présente également l'avantage d'être aisément hydratable en surface en contact avec de l'eau ou une surface humide au moment de l'utilisation.
Enfin, cette composition est très agréable au toucher et présente une douceur remarquable.

La composition selon l'invention comprend donc, dans un milieu adéquat c'est-à-dire par exemple un milieu cosmétiquement ou pharmaceutiquement acceptable, un agent structurant qui est de préférence insoluble dans ledit milieu.
Cet agent structurant présente la particularité de s'éliminer facilement du support sur lequel est appliquée la composition, et en particulier des cheveux ou de la peau, par simple dilution.
Cet agent structurant permet de préparer une composition se présentant sous forme solide, mais qui peut également être sous forme solide déformable. On entend par 'déformable' le fait que la composition se présente sous une forme solide, sèche, malléable, ressemblant à de la guimauve (voir le document US-A-3 682 659 pour la consistance de la guimauve).
La composition, dans ce cas, peut être modelée aisément à la main, comme de la pâte à modeler pour enfants. Elle peut également être rompue facilement à la main afin de ne prélever que la quantité nécessaire de produit. En particulier, cette composition peut être conditionnée sous forme de monodose et par exemple sous forme de petits cubes ou de berlingots.
La composition selon l'invention peut présenter notamment une structure homogène, même lorsqu'elle comprend des constituants conduisant normalement à des phases distinctes (constituants non miscibles sans emploi de tensioactif, tels que huile et eau; constituants difficiles à mettre en oeuvre ensemble tels que polymère cationique et polymère anionique). Ceci est en particulier vrai lorsque la composition est préparée par extrusion.

L'agent structurant présent dans la composition est de préférence formé de particules rigides et est insoluble dans le milieu. Il peut être formé d'un ou plusieurs types de particules.
En vue d'obtenir un solide au toucher agréable et doux, il est souhaitable d'utiliser des particules ayant une granulométrie moyenne de 1 à 300 microns (µm), par exemple de 5 à 200 µm, et de préférence de 10 à 100 µm, et encore mieux de 15 à 40 µm.
Afin de conférer à la composition de l'invention un aspect aéré et léger, on utilise avantageusement des particules ayant une densité inférieure à 0,1, notamment inférieure à 0,09 et, mieux, inférieure à 0,06 et, encore mieux inférieure à 0,04.
En vue d'obtenir cette faible densité, on utilise avantageusement des particules creuses remplies d'un gaz. Ce gaz peut être notamment de l'air, de l'azote, de l'isobutane, de l'isopentane.
Selon une autre caractéristique avantageuse de l'invention, les particules se présentent sous forme de billes. Il est toutefois possible d'utiliser des particules ayant la forme de fibres ou d'aiguilles.
Ces particules peuvent être réalisées en différents matériaux inertes ne réagissant pas chimiquement avec le milieu; en particulier ces particules ne réagissent pas avec les huiles, les tensioactifs, l'eau et les différents autres constituants de la composition tels que les actifs.

Comme critère de choix de l'agent structurant, on peut réaliser le test suivant :
- mélange des particules testées avec de l'eau contenant un colorant usuel, jusqu'à l'obtention d'une pâte colorée,
- versement d'une goutte d'eau sur la pâte ainsi préparée.
Lorsque la pâte au point d'impact de la goutte d'eau est beaucoup plus claire que le reste de la pâte, cela signifie que les particules considérées sont candidates comme agent structurant; inversement, lorsque la pâte au point d'impact ne s'est pas décolorée, les particules considérées ne sont pas appropriées.

Les particules inertes sont avantageusement réalisées en verre ou en matériaux thermoplastiques comme les polyamides tels que le Nylon, les polymères ou copolymères d'acrylonitrile, de chlorure de vinylidène, de chlorure de vinyle et/ou de monomère acrylique ou styrénique, éventuellement expansés. Le monomère acrylique est par exemple un acrylate ou méthacrylate de méthyle ou d'éthyle. Le monomère styrénique est par exemple l'α-méthyl-styrène ou le styrène.
Comme particules de verre utilisables dans l'invention, on peut citer les billes de verres creuses vendues par la société 3M sous la référence Scotchlite Glass Bubbles S 22 ®. 95 % de ces billes ont un diamètre inférieur à 74 µm.
Comme particules de Nylon®, on peut utiliser les particules d'Orgasol® vendues par la société Atochem. Ces particules sont des sphères pleines, poreuses, de diamètre allant de 5 µm à 60 µm.
De préférence, les particules sont des particules creuses déformables d'un copolymère expansé de chlorure de vinylidène et d'acrylonitrile, ou de chlorure de vinylidène, d'acrylonitrile et de méthacrylate. On peut par exemple utiliser un polymère contenant 0-60% de motifs dérivés du chlorure de vinylidène, 20-90% de motifs dérivés d'acrylonitrile et 0-50% de motifs dérivés d'un monomère acrylique ou styrénique, la somme des pourcentages (en poids) étant égale à 100%. Le monomère acrylique peut être le (méth)acrylate de méthyle ou d'éthyle. Le monomère styrénique peut être le styrène ou le α-méthyl-styrène.
Plus préférentiellement, les particules utilisées dans la présente invention sont des particules creuses d'un copolymère expansé de chlorure de vinylidène et d'acrylonitrile ou de chlorure de vinylidène, d'acrylonitrile et de méthacrylate de méthyle. Ces particules peuvent être sèches ou hydratées.
De façon préférentielle, la masse volumique de ces particules est choisie dans la gamme allant de 15 à 200 kg/m³ et mieux de 40 à 120 kg/m³, et encore mieux de 60 à 80 kg/m³.
Les particules utilisables dans l'invention sont par exemple les microsphères de terpolymère expansé de chlorure de vinylidène, d'acrylonitrile et de méthacrylate, vendues sous la marque EXPANCEL ® par la société Nobel Casco et en particulier sous les références 551 DE 12 (granulométrie d'environ 12 µm et masse volumique d'environ 40 kg/m³), 551 DE 20 (granulométrie d'environ 30 µm et masse volumique d'environ 65 kg/m³), 551 DE 50 (granulométrie d'environ 40 µm), 461 DE 50 et 642 WE 50 de 50 µm de granulométrie environ, 551 DE 80 (granulométrie de 80 µm environ). On peut aussi utiliser des particules de ce même terpolymère expansé, ayant une granulométrie d'environ 18 µm et une masse volumique d'environ 60 à 80 kg/m³ (Expancel ® EL23) ou encore de granulométrie d'environ 34 µm et de masse volumique d'environ 20 kg/m³. On peut encore utiliser des particules de polymère de chlorure de vinylidène et d'acrylonitrile ou de chlorure de vinylidène, d'acrylonitrile et de méthacrylate non expansé comme celles vendues sous la marque EXPANCEL ® avec la référence 551 DU 10 (granulométrie d'environ 10 µm) ou 461 DU 15 (granulométrie d'environ 15 µm).
Comme autres particules creuses polymériques utilisables dans l'invention, on peut encore citer les polymères et les copolymères obtenus à partir des esters ou acides, itaconique, citraconique, maléique, fumarique, de l'acétate ou lactate de vinyle. (Voir à cet effet le document JP-A-2-112304).

On peut encore citer les microsphères microporeuses vendues par Dow Corning sous la dénomination POLYTRAP ® qui sont formées de copolymères méthacrylate de lauryle/diméthacrylate d'éthylèneglycol; ou celles vendues par Seppic sous la dénomination MICROPEARL ®; ou les microparticules de silice à porosités ouvertes ou creuses telles que celles vendues par Miyoshi Kasei sous la dénomination 'SILICA BEADS S700'.

La composition selon l'invention peut comprendre, d'une manière générale, 1 à 50% en poids d'agent structurant, de préférence 5 à 10% en poids par rapport au poids total de la composition.

La composition comprend en outre au moins un agent absorbant organique. Par agent absorbant, on entend tout composé susceptible de piéger rapidement une grande quantité d'eau. L'agent absorbant organique est donc généralement un composé hydrophile ou amphiphile.
Par agent absorbant au sens de la présente invention, on entend tout composé ayant une capacité statique d'absorption d'eau, à température ambiante (25°C), supérieure ou égale à 3 fois son poids.
De préférence, on choisit les agents absorbants parmi les composés ayant une capacité statique d'absorption d'eau supérieure ou égale à 5 fois son poids, et préférentiellement supérieure ou égale à 15 fois son poids.
Le test pour mesurer ladite capacité statique d'absorption d'eau est décrit avant les exemples.
Cet agent absorbant permet d'obtenir une composition sous forme de solide, notamment déformable, qui se délite aisément à l'aide d'un diluant qui est généralement de l'eau, froide ou chaude, mais qui peut également être de l'eau additionnée d'un ou plusieurs solvants polaires cosmétiquement acceptables, tels que les alcools ayant 2 à 20 atomes de carbone (isopropanol, éthanol notamment), le propylène glycol, ou encore de l'eau additionnée d'un ou plusieurs tensioactifs. On peut aussi utiliser des milieux aqueux plus complexes.

En particulier, on peut choisir l'agent absorbant parmi, seul ou en mélange :
- les carboxyméthylcelluloses de sodium réticulées.
De tels produits sont notamment vendus par AVEBE sous la dénomination PRIMELLOSE.
- les sciures et les farines de bois de granulométrie moyenne inférieure à 250 microns, et notamment la farine d'épicéa ou la farine de hêtre.
De tels produits sont notamment vendus par la Société Parisienne des Sciures, sous la dénomination T140 (farine d'épicéa) ou H160/0 (farine de hêtre).
- les amidons modifiés.
Les amidons naturels ne possèdent généralement pas une bonne capacité statique d'absorption d'eau; il est généralement nécessaire de les modifier de manière à obtenir un agent absorbant au sens de l'invention. Une telle modification peut consister en un greffage de sel de sodium faiblement réticulé et/ou en une prégélatinisation.
Parmi les amidons modifiés susceptibles d'être utilisés, on peut citer les fécules de pomme de terre prégélatinisées quatemisées, les amidons de maïs prégélatinisés, les carboxyméthylamidons de pomme de terre réticulés, les phosphates de diamidon de manioc prégélatinisés et éventuellement hydroxypropylés, les phosphates de diamidon de pomme de terre prégélatinisés et éventuellement acétylés. Parmi les produits commercialement disponibles, on peut citer les produits vendus par AVEBE sous les dénominations PREGEL ou PRIMOGEL, ou ceux vendus par NATIONAL STARCH sous la dénomination STRUCTURE ZEA.
- certains polyacrylates, et notamment ceux vendus par OSAKA YUKI sous la dénomination PQ POLYMER.

Les agents absorbants particulièrement préférés sont notamment choisis parmi les farines de bois et les amidons modifiés.

La composition selon l'invention peut comprendre, d'une manière générale, 1 à 60% en poids d'agent absorbant organique, de préférence 5 à 50% en poids, et préférentiellement 10 à 35% en poids, par rapport au poids total de la composition.
Cette quantité peut varier selon la nature de l'agent absorbant utilisé ainsi que selon la facilité de délitage souhaitée.
Ainsi, les composés dont la structure chimique comprend au moins un motif dérivé du sucre sont généralement employés en une quantité supérieure ou égale à 5% en poids par rapport au poids total de la composition.

La composition selon l'invention comprend en outre un milieu adéquat pour l'utilisation envisagée, en particulier un milieu cosmétiquement ou pharmaceutiquement acceptable.
Ce milieu est de préférence aqueux, c'est-à-dire qu'il comprend soit uniquement de l'eau, soit de l'eau et un solvant tel que par exemple l'éthanol, le propylène glycol, le butylène glycol, l'isopropanol, les éthers de glycol tel que les alkyl(C₁-C₄)éther de mono, di- ou tripropylène glycol, mono, di- ou triéthylène glycol, le dipropylène glycol, le diéthylène glycol et leurs mélanges.
Le milieu peut également être anhydre ou essentiellement anhydre.

La composition selon l'invention peut en outre comprendre tout additif susceptible d'être utilisé dans le domaine d'application considéré.
En particulier, elle peut comprendre des huiles minérales, animales, végétales, synthétiques ou siliconées, lesdites huiles pouvant être volatiles ou non; des corps gras pâteux; des cires d'origine animale, minéral, végétale ou synthétique; des gélifiants; des filtres UV; des parfums; des tensioactifs non ioniques, anioniques, cationiques ou amphotères; des polymères éventuellement filmogènes; des conservateurs; des antioxydants; des agents régulateurs de pH; des séquestrants; des pigments; des charges; des actifs cosmétiques ou pharmaceutiques; des générateurs d'eau oxygénée et/ou d'oxygène; de l'ammoniaque; des sels d'ammonium; des agents anti-radicaux libres; des hydratants; des agents réducteurs; des silicones.
Bien entendu l'homme du métier veillera à choisir ce ou ces éventuels additifs complémentaires, et/ou leur quantité, de manière telle que les propriétés avantageuses de la composition selon l'invention ne soient pas, ou substantiellement pas, altérées par l'adjonction envisagée.

D'une manière préférée, la composition finale obtenue peut présenter une densité allant de 0,03 à 1, avec une teneur en eau pouvant être comprise entre 0 et 98% en poids. Il est également possible de déshydrater, partiellement ou totalement, la composition préparée selon l'invention.
En effet, on a constaté que le délitage pouvait être lié, d'une part, à la nature et/ou la quantité d'agent absorbant présent, et, d'autre part, à la quantité d'eau présente dans la composition. L'eau initialement présente étant absorbée, au sein de la composition, par l'agent absorbant, il s'en suit que la capacité d'absorption dudit agent est amoindrie, d'où un délitage moins facile.
De plus, le fait de disposer d'une composition anhydre, peut permettre de diminuer la quantité d'agent conservateur présent dans la formule, voire de préparer une composition ne comprenant pas du tout de conservateur.

La composition selon l'invention trouve en particulier une application dans le domaine cosmétique, notamment dans le domaine capillaire et dans le domaine du nettoyage et/ou du soin de la peau du corps et/ou du visage.
La composition selon l'invention peut se présenter sous forme de bâton, de crayon, de stick, de pain, voire de pâte, et constituer en elle-même un nouveau type de produit cosmétique ou pharmaceutique.
En particulier, elle peut être un produit de nettoyage, de soin et/ou de conditionnement des cheveux tel que des soins capillaires à rincer ou non, des produits de coiffage; des shampooings ou après shampooings.
Elle peut également être un produit de nettoyage, de soin et/ou d'hygiène de la peau humaine (corps et/ou visage), des muqueuses et/ou du cuir chevelu, et notamment un produit pour le bain ou la douche, un déodorant, un produit de soin du visage, un masque pour le visage éventuellement chauffant, un produit de nettoyage du corps ou du visage, un amincissant, un produit de rasage ou d'après-rasage, un produit parfumant.
Elle peut encore être un produit de maquillage tel que rouge à lèvres, mascaras, fond de teint ou fard à joues ou à paupières.

Par exemple, lorsqu'elle se présente sous la forme d'un masque, éventuellement chauffant, à appliquer sur le visage, la composition selon l'invention peut comprendre :
- 2 à 25% en poids, de préférence 5 à 15% en poids, d'agent structurant;
- 5 à 40% en poids, de préférence 10 à 25% en poids, d'agent absorbant;
- 5 à 40% en poids, de préférence 15 à 30% en poids, de kaolin;
- 10 à 70% en poids, de préférence 20 à 50% en poids, de glycol,
- 2 à 40% en poids, de préférence 5 à 25% en poids, de tensioactif, et
- 0 à 50% en poids, de préférence 15 à 30% en poids d'eau.

Lorsqu'elle se présente sous la forme d'un stick poudreux à réhydrater ou non, la composition selon l'invention peut comprendre :
- 2 à 25% en poids, de préférence 5 à 15% en poids, d'agent structurant;
- 5 à 35% en poids, de préférence 10 à 25% en poids, d'agent absorbant;
- 20 à 80% en poids, de préférence 30 à 60% en poids, d'huile;
- 0 à 60% en poids, de préférence 10 à 50% en poids, de glycol,
- 2 à 30% en poids, de préférence 10 à 20% en poids, de tensioactif, et
- 0 à 30% en poids, de préférence 10 à 20% en poids d'eau.

La composition selon l'invention peut être préparée par tout moyen connu de l'homme du métier, et en particulier par simple mélange des différents constituants et moulage dans un moule adéquat. Elle peut encore être par mélange suivi de malaxage et extrusion dans un extrudeur, de préférence un extrudeur bi-vis, notamment un extrudeur bi-vis telle que celles décrites dans les brevets EP605284 ou FR2715306, et dans laquelle les deux vis tournent dans le même sens.
La masse extrudée sort de la filière d'extrusion sous la forme de boudins de diamètre donné selon la filière utilisée, pouvant être ensuite découpés et mis en forme, notamment, de bâton ou de pain solide. D'autres formes peuvent bien entendu être réalisées en choisissant des filières appropriées et des dispositifs de mise en forme des produits finaux adaptés à la forme recherchée.
La masse extrudée peut également être déshydratée et/ou broyée et/ou compactée après son obtention.
Le procédé d'extrusion peut être effectué à chaud, à température ambiante ou en présence d'un système de réfrigération. De préférence la totalité du procédé d'extrusion est réalisé à température ambiante, de l'ordre de 20-25°C, ou à froid, ce qui permet l'utilisation de matières premières sensibles à la chaleur, du type vitamines ou huiles volatiles.
D'autre part, ceci permet d'introduire les matières premières sensibles à la chaleur dans n'importe quelle zone de l'extrudeur (en tête, au milieu ou en final) puisqu'aucune détérioration due à la chaleur n'est à craindre. Ceci est en particulier avantageux pour l'introduction des agents structurant du type EXPANCEL.

Il est également possible d'effectuer une partie de l'extrusion sous gaz inerte (azote par exemple), ce qui peut être avantageux lorsque l'on emploie des produits oxydables.
L'extrusion étant généralement effectuée à température ambiante, la matrice formant la composition n'est pas une matrice d'un réseau expansé.

L'invention est illustrée par les exemples donnés ci-après. Dans ces exemples, les pourcentages sont donnés en poids et MA signifie matière active.

### Test de mesure de la capacité statique d'absorption d'eau

A température ambiante, on dispose dans un bécher le composé à tester en une quantité de x grammes; on ajoute de l'eau en une quantité de 3x grammes. On laisse reposer, sans agiter, pendant 1 minute.
S'il ne reste plus d'eau libre (eau surnageante) après ladite minute, le composé peut être considéré comme un agent absorbant au sens de l'invention.

### Test de délitage (délitage dynamique)

A température ambiante (environ 25°C), on dispose 4 grammes de masse extrudée sous forme de boudin de diamètre d'environ 2,5 cm (environ 25 cm³ à titre indicatif) dans un récipient de 12 cm de diamètre et 5 cm de hauteur.
On ajoute 100 ml d'eau et on mélange manuellement à l'aide d'une spatule.

On considère que la composition se délite au sens de l'invention lorsqu'il ne reste plus d'agrégats non dissociés après 120 secondes (2 minutes).
On considère que le délitage est rapide lorsqu'il ne reste plus d'agrégats non dissociés après 20 secondes.

### Exemple 1

On teste différentes matières premières selon le test de mesure de capacité d'absorption statique d'eau ci-dessus mentionné, et l'on obtient les résultats suivants:

| Nature chimique | Produit commercial | Résultat |
|---|---|---|
| Adipate de diamidon de maïs cireux acétylé réticulé | C*TEX 06205 de Cerestar | Non |
| Adipate de diamidon de maïs cireux acétylé réticulé | C*TEX 063004 de Cerestar | Non |
| Amidon de maïs prégélatinisé | C*PHARM 12018 de Cerestar | Oui |
| Carboxyméthylamidon de pomme de terre, sel de sodium, réticulé | PRIMOGEL de Avebe | Oui |
| Fécule de pomme de terre prégélatinisée quaternisée | SOLVITOSE N de Avebe | Oui |
| Maltodextrine de pomme de terre hydrolysée (DE 3) acétylée | AMYLOGUM CLS de Avebe | Non |
| Phosphate de diamidon de manioc hydroxypropylé prégélatinisé | PREGEL VA-70-T de Avebe | Oui |
| Phosphate de diamidon de manioc prégélatinisé | PREGEL TK1 de Avebe | Oui |
| Phosphate de diamidon de pomme de terre acétylé prégélatinisé | PREJEL 200 de Avebe | Oui |
| Carbomer | CARBOPOL 980 de Goodrich | Non |
| Dérivé de cellulose | Natrosol Plus Grade 330CS de Aqualon | Non |
| 'Non' signifie que la matière première ne peut pas être considérée comme un agent absorbant au sens de l'invention. | | |
| 'Oui' signifie que la matière première est un agent absorbant au sens de l'invention. | | |

### Exemple 2

On a réalisé, à partir d'un même support (eau + propylène glycol + tensioactif anionique), une formule comprenant uniquement un agent structurant (formule A témoin) et une formule comprenant un agent structurant et un agent absorbant (formule B selon l'invention).
Chaque formule a été placée dans un bécher en présence de la même quantité d'eau et mise sous agitation manuelle.
Agent structurant : Expancel 551 DE 50
Agent absorbant : Farine d'épicéa T140
Quantité d'eau : 80 ml pour 20 g de formule

On observe la facilité à se déliter pour les deux formules testées. On obtient les résultats suivants :

| | Formule A | Formule B |
|---|---|---|
| Agent structurant | 5% en poids | 5% en poids |
| Agent absorbant | 0 | 25% en poids |
| Délitage | difficile | aisé |

On constate donc que la présence d'agent absorbant permet bien d'améliorer le délitage d'une composition comprenant un agent structurant.

### Exemple 3

On prépare une composition de soin capillaire à rincer comprenant les ingrédients suivants :
- Carboxyméthylamidon de pomme de terre réticulé 27%
- Farine de bois d'épicéa 22%
- Microsphères expansées de copolymère 5% acrylonitrile/méthacrylate de méthyle
   (Expancel 551 DE 50 de Nobel Casco)
- Propylène glycol
- Chlorure de cétyl triméthyl ammonium 4% MA
- Copolymère de chlorure de diméthyldiallyl ammonium/ acide acrylique 2,7% MA
- Eau qsp 100%

La composition est préparée à l'aide d'un extrudeur de type BC21 de la société CLEXTRAL.
Les matières premières sont introduites, à l'entrée de l'extrudeur bi-vis, dans la zone d'alimentation à température ambiante, de préférence environ 20°C. De préférence on introduit les matières premières solides en tête de l'extrudeur puis les matières premières liquides sont introduites latéralement. Le tout est malaxé dans diverses zones de l'extrudeur à température ambiante; la masse obtenue est transportée vers la sortie de l'extrudeur et extrudée au travers d'une filière. La vitesse des vis est de préférence comprise entre 400 et 500 tours/minute.

On obtient une masse extrudée sous la forme d'un boudin de 2,5 cm de diamètre. Cette composition se délite aisément par mélange dans un bol avec de l'eau et peut alors être directement utilisée.

### Exemple 4

On prépare, selon le mode opératoire de l'exemple 3, une composition pour la douche comprenant les ingrédients suivants:
- Carboxyméthylamidon de pomme de terre 34 %
- Microsphères expansées de copolymère 4,5 %
   acrylonitrile/méthacrylate de méthyle
- Lauryl éther sulfate de sodium 40 % MA
- Gommes (Caroube et carraghénane) 3,5%
- Eau qsp 100 %

On obtient une composition finale extrudée qui se délite aisément lorsqu'elle est appliquée sur une peau mouillée.

### Exemple 5

On prépare un produit nettoyant comprenant les ingrédients suivants:
- Carboxyméthylamidon de pomme de terre 16 %
- Microbilles de silice poreuse 4 %
- Lauryl glutamate monosodique 8 %
- Propylène glycol 10 %
- Cire liquide de jojoba 5 %
- Polydécène hydrogéné qsp 100 %

On obtient un nettoyant pour le visage, à réhydrater avant utilisation.

## Revendications

1. Composition cosmétique ou pharmaceutique se présentant sous forme de solide, pouvant être déformable, et comprenant, dans un milieu cosmétiquement ou pharmaceutiquement acceptable, au moins un agent structurant, **caractérisée par le fait qu'**elle comprend en outre 1 à 60% en poids d'au moins un agent absorbant organique ayant une capacité statique d'absorption d'eau, à température ambiante (25°C), supérieure ou égale à 3 fois son poids, choisi parmi les carboxyméthylcelluloses de sodium réticulées; les sciures et les farines de bois de granulométrie moyenne inférieure à 250 microns et notamment la farine d'épicéa ou la farine de hêtre; les amidons modifiés par greffage de sel de sodium faiblement réticulé et/ou par prégélatinisation; leurs mélanges.

2. Composition selon la revendication 1, dans laquelle l'agent absorbant est choisi parmi les fécules de pomme de terre prégélatinisées quatemisées, les amidons de maïs prégélatinisés, les carboxyméthylamidons de pomme de terre réticulés, les phosphates de diamidon de manioc prégélatinisés et éventuellement hydroxypropylés, les phosphates de diamidon de pomme de terre prégélatinisés et éventuellement acétylés.

3. Composition selon l'une des revendications précédentes, se présentant sous forme solide déformable.

4. Composition selon l'une des revendications précédentes, dans laquelle l'agent structurant est formé de particules ayant une granulométrie moyenne de 1 à 300 microns (µm), par exemple de 5 à 200 µm, de préférence de 10 à 100 µm, et encore mieux de 15 à 40 µm.

5. Composition selon l'une des revendications précédentes, dans laquelle l'agent structurant est formé de particules ayant une densité inférieure à 0,1, notamment inférieure à 0,09 et, mieux, inférieure à 0,06 et, encore mieux inférieure à 0,04.

6. Composition selon l'une des revendications précédentes, dans laquelle l'agent structurant est choisi parmi les billes de verre, les particules de matériaux thermoplastiques comme les polyamides tels que le Nylon, les polymères ou copolymères d'acrylonitrile, de chlorure de vinylidène, de chlorure de vinyle et/ou de monomère acrylique ou styrénique, éventuellement expansés; les microsphères microporeuses; les microparticules de silice.

7. Composition selon l'une des revendications précédentes, dans laquelle l'agent structurant est choisi parmi les particules creuses d'un copolymère expansé de chlorure de vinylidène et d'acrylonitrile, ou de chlorure de vinylidène, d'acrylonitrile et de méthacrylate de méthyle.

8. Composition selon l'une des revendications précédentes, dans laquelle l'agent structurant est présent à raison de 1 à 50% en poids, de préférence de 5 à 10% en poids, par rapport au poids total de la composition.

9. Composition selon l'une des revendications précédentes, dans laquelle l'agent absorbant a une capacité statique d'absorption d'eau, à température ambiante (25°C), supérieure ou égale à 5, et préférentiellement supérieure ou égale à 15.

10. Composition selon l'une des revendications précédentes, dans laquelle l'agent absorbant est présent en une quantité de 5 à 50% en poids, préférentiellement 10 à 35% en poids, par rapport au poids total de la composition.

11. Composition selon l'une des revendications précédentes, dans laquelle le milieu cosmétiquement ou pharmaceutiquement acceptable comprend uniquement de l'eau; comprend de l'eau et un solvant; ou est anhydre ou essentiellement anhydre.

12. Composition selon l'une des revendications précédentes, se présentant sous la forme d'un produit de nettoyage, de soin et/ou de conditionnement des cheveux tel que des soins capillaires à rincer ou non, des produits de coiffage; des shampooings ou après shampooings; d'un produit de nettoyage, de soin et/ou d'hygiène de la peau humaine (corps et/ou visage), des muqueuses et/ou du cuir chevelu, et notamment un produit pour le bain ou la douche, un déodorant, un produit de soin du visage, un masque pour le visage éventuellement chauffant, un produit de nettoyage du corps ou du visage, un amincissant, un produit de rasage ou d'après-rasage, un produit parfumant; d'un produit de maquillage tel que rouge à lèvres, mascaras, fond de teint ou fard à joues ou à paupières.

13. Composition selon l'une des revendications précédentes, susceptible d'être préparée par mélange suivi de malaxage et extrusion dans un extrudeur, de préférence un extrudeur bi-vis.

14. Composition selon la revendication 13, dans laquelle l'extrusion est réalisée à température ambiante, de l'ordre de 20-25°C, ou à froid.

15. Composition selon la revendication 1, se présentant sous la forme d'un masque, éventuellement chauffant, à appliquer sur le visage, et comprenant :
- 2 à 25% en poids, de préférence 5 à 15% en poids, d'agent structurant;
- 5 à 40% en poids, de préférence 10 à 25% en poids, d'agent absorbant;
- 5 à 40% en poids, de préférence 15 à 30% en poids, de kaolin;
- 10 à 70% en poids, de préférence 20 à 50% en poids, de glycol,
- 2 à 40% en poids, de préférence 5 à 25% en poids, de tensioactif, et
- 0 à 50% en poids, de préférence 15 à 30% en poids d'eau.

16. Composition selon la revendication 1, se présentant sous la forme d'un stick poudreux à réhydrater ou non, et comprenant :
- 2 à 25% en poids, de préférence 5 à 15% en poids, d'agent structurant;
- 5 à 35% en poids, de préférence 10 à 25% en poids, d'agent absorbant;
- 20 à 80% en poids, de préférence 30 à 60% en poids, d'huile;
- 0 à 60% en poids, de préférence 10 à 50% en poids, de glycol,
- 2 à 30% en poids, de préférence 10 à 20% en poids, de tensioactif, et
- 0 à 30% en poids, de préférence 10 à 20% en poids d'eau.

17. Utilisation d'une composition selon l'une quelconque des revendications précédentes pour le traitement cosmétique de la peau du visage et du corps, des cheveux, du cuir chevelu ou des muqueuses.

## Claims

1. Cosmetic or pharmaceutical composition in the form of a solid, which can be deformed, the said composition comprising, in a cosmetically or pharmaceutically acceptable medium, at least one structuring agent, **characterized in that** it additionally comprises 1 to 60% by weight of at least one organic absorbent having a static water absorption capacity at room temperature (25°C) of greater than or equal to 3 times its weight and is selected from crosslinked sodium carboxymethylcelluloses; sawdusts and wood flours with an average particle size of less than 250 microns, and especially spruce flour or beech flour; starches modified by grafting of a slightly crosslinked sodium salt and/or by pregelatinization; and mixtures thereof.

2. Composition according to Claim 1, in which the absorbent is selected from quaternized pregelatinized potato starches, pregelatinized cornstarches, crosslinked potato carboxymethylstarches, pregelatinized and optionally hydroxypropylated manioc di-starch phosphates, and pregelatinized and optionally acetylated potato di-starch phosphates.

3. Composition according to either of the preceding claims, which is in the form of a deformable solid.

4. Composition according to one of the preceding claims, in which the structuring agent is formed of particles having an average size of from 1 to 300 microns (µm), for example from 5 to 200 µm, preferably from 10 to 100 µm and, better still from 15 to 40 µm.

5. Composition according to one of the preceding claims, in which the structuring agent is formed of particles having a density of less than 0.1, in particular less than 0.09 and, more preferably, less than 0.06 and, better still, less than 0.04.

6. Composition according to one of the preceding claims, in which the structuring agent is selected from glass beads, particles of thermoplastics such as polyamides, for instance nylon, polymers or copolymers of acrylonitrile, of vinylidene chloride, of vinyl chloride and/or of acrylic or styrenic monomer, expanded or otherwise; microporous microspheres; and silica microparticles.

7. Composition according to one of the preceding claims, in which the structuring agent is selected from hollow particles of an expanded vinylidene chloride and acrylonitrile copolymer or of an expanded vinylidene chloride, acrylonitrile and methyl methacrylate copolymer.

8. Composition according to one of the preceding claims, in which the structuring agent is present in an amount of from 1 to 50% by weight, preferably from 5 to 10% by weight, relative to the total weight of the composition.

9. Composition according to one of the preceding claims, in which the absorbent has a static water absorption capacity at room temperature (25°C) of greater than or equal to 5, and, preferably, greater than or equal to 15.

10. Composition according to one of the preceding claims, in which the absorbent is present in an amount of from 5 to 50% by weight and, preferably, from 10 to 35% by weight, relative to the total weight of the composition.

11. Composition according to one of the preceding claims, in which the cosmetically or pharmaceutically acceptable medium comprises solely water; comprises water and a solvent; or is anhydrous or essentially anhydrous.

12. Composition according to one of the preceding claims, which is in the form of a product for cleansing, caring for and/or conditioning the hair, such as rinse-out or leave-in hair-care products, hair-styling products; shampoos or conditioners; a cleansing, care and/or hygiene product for the human skin (body and/or face), mucosae and/or scalp, and especially a bath or shower product, a deodorant, a facial care product, a warming or non-warming face mask, a body or face cleansing product, a slimming product, a shaving or after-shave product, a perfume; or a make-up product such as lipsticks, mascaras, foundations, blushers or eyeshadows.

13. Composition according to one of the preceding claims, preparable by mixing followed by kneading and extrusion in an extruder, preferably a twinscrew extruder.

14. Composition according to Claim 13, in which extrusion is carried out at room temperature, of the order of 20-25°C, or under cold conditions.

15. Composition according to Claim 1, in the form of a warming or non-warming mask for application to the face and comprising:
- 2 to 25% by weight, preferably 5 to 15% by weight, of structuring agent;
- 5 to 40% by weight, preferably 10 to 25% by weight, of absorbent;
- 5 to 40% by weight, preferably 15 to 30% by weight, of kaolin;
- 10 to 70% by weight, preferably 20 to 50% by weight, of glycol;
- 2 to 40% by weight, preferably 5 to 25% by weight, of surfactant; and
- 0 to 50% by weight, preferably 15 to 30% by weight, of water.

16. Composition according to Claim 1, in the form of a powder stick, for rehydration or otherwise, and comprising:
- 2 to 25% by weight, preferably 5 to 15% by weight, of structuring agent;
- 5 to 35% by weight, preferably 10 to 25% by weight, of absorbent;
- 20 to 80% by weight, preferably 30 to 60% by weight, of oil;
- 0 to 60% by weight, preferably 10 to 50% by weight, of glycol;
- 2 to 30% by weight, preferably 10 to 20% by weight, of surfactant; and
- 0 to 30% by weight, preferably 10 to 20% by weight, of water.

17. Use of a composition according to any one of the preceding claims for the cosmetic treatment of the skin of the face and of the body, the hair, the scalp or the mucosae.

## Patentansprüche

1. Kosmetische oder pharmazeutische Zusammensetzung, die in Form eines Feststoffes vorliegt, der verformbar sein kann, und die in einem kosmetisch oder pharmazeutisch akzeptablen Medium mindestens ein Texturierungsmittel enthält, **dadurch gekennzeichnet, dass** sie ferner 1 bis 60 Gew.-% mindestens eines organischen Absorptionsmittels enthält, das bei Umgebungstemperatur (25 °C) eine statische Wasseraufnahmefähigkeit des 3fachen seines Gewichts oder darüber aufweist und das unter den vernetzten Natriumcarboxymethylcellulosen; Sägemehlen und Holzmehlen mit einer mittleren Korngröße unter 250 µm und insbesondere Fichtenmehl oder Buchenmehl; durch Pfropfen der leicht vernetzten Natriumsalze und/oder durch Verkleisterung modifizierten Stärkeverbindungen; und deren Gemischen ausgewählt ist.

2. Zusammensetzung nach Anspruch 1, wobei das Absorptionsmittel unter den quaternisierten, verkleisterten Stärkeverbindungen aus Kartoffeln, verkleisterten Maisstärken, vernetzten Carboxymethylstärkeverbindungen aus Kartoffeln, verkleisterten Dimaniokstärkephosphaten, die gegebenenfalls hydroxypropyliert sind, und verkleisterten Dikartoffelstärkephosphaten, die gegebenenfalls acetyliert sind, ausgewählt ist.

3. Zusammensetzung nach einem der vorhergehenden Ansprüche, die in Form eines verformbaren Feststoffes vorliegt.

4. Zusammensetzung nach einem der vorhergehenden Ansprüche, wobei das Texturierungsmittel aus Partikeln mit einer mittleren Korngröße von 1 bis 300 Mikrometern (µm), beispielsweise 5 bis 200 µm, vorzugsweise 10 bis 100 µm und besser 15 bis 50 µm gebildet wird.

5. Zusammensetzung nach einem der vorhergehenden Ansprüche, wobei das Texturierungsmittel aus Partikeln mit einer Dichte unter 0,1, insbesondere unter 0,09, besser unter 0,06 und noch besser unter 0,04 gebildet wird.

6. Zusammensetzung nach einem der vorhergehenden Ansprüche, wobei das Texturierungsmittel unter den Glaskugeln, Partikeln aus thermoplastischen Materialien, wie Polyamiden, beispielsweise Nylon, Polymeren oder Copolymeren von Acrylnitril, Vinylidenchlorid, Vinylchlorid und/oder einem Acrylmonomer oder Styrolmonomer, die gegebenenfalls expandiert sind; mikroporösen Mikrosphären; und Siliciumdioxidmikropartikeln ausgewählt ist.

7. Zusammensetzung nach einem der vorhergehenden Ansprüche, wobei das Texturierungsmittel unter den Hohlpartikeln eines expandierten Copolymers von Vinylidenchlorid und Acrylnitril oder Vinylidenchlorid, Acrylnitril und Methylmethacrylat ausgewählt ist.

8. Zusammensetzung nach einem der vorhergehenden Ansprüche, wobei das Texturierungsmittel in einer Menge von 1 bis 50 Gew.-% und vorzugsweise 5 bis 10 Gew.-%, bezogen auf das Gesamtgewicht der Zusammensetzung, enthalten ist.

9. Zusammensetzung nach einem der vorhergehenden Ansprüche, wobei das Absorptionsmittel bei Umgebungstemperatur (25 °C) eine statische Wasseraufnahmefähigkeit von 5 oder darüber und vorzugsweise mindestens 15 aufweist.

10. Zusammensetzung nach einem der vorhergehenden Ansprüche, wobei das Absorptionsmittel in einer Menge von 5 bis 50 Gew.-% und vorzugsweise 10 bis 35 Gew.-%, bezogen auf das Gesamtgewicht der Zusammensetzung, enthalten ist.

11. Zusammensetzung nach einem der vorhergehenden Ansprüche, wobei das kosmetisch oder pharmazeutisch akzeptable Medium ausschließlich Wasser enthält; Wasser und ein Lösungsmittel enthält; oder wasserfrei oder im Wesentlichen wasserfrei ist.

12. Zusammensetzung nach einem der vorhergehenden Ansprüche, die in Form eines Produkts zur Reinigung, zur Pflege und/oder zur Konditionierung der Haare, wie Haarpflegeprodukte, die ausgespült werden oder im Haar verbleiben, Produkte für die Frisurgebung; Haarwaschmittel oder Produkte zur Anwendung nach der Haarwäsche; in Form eines Produkts zur Reinigung, zur Pflege und/oder für die Hygiene der menschlichen Haut (Körper und/oder Gesicht), der Schleimhäute und/oder der Kopfhaut, wie insbesondere Produkte für das Bad oder die Dusehe, Deodorants, Produkte zur Pflege des Gesichts, Masken für das Gesicht, die gegebenenfalls Wärme entwickeln, Produkte zur Reinigung des Körpers oder des Gesichts, schlanker machende Produkte, Produkte für die Rasur, nach der Rasur anzuwendende Produkte, parfümierende Produkte; oder in Form eines Produktes zum Schminken vorliegt, wie Lippenstifte, Mascaras, Make-up, Wangenrouge oder Lidschatten.

13. Zusammensetzung nach einem der vorhergehenden Ansprüche, die durch Mischen und anschließendes Kneten und Extrudieren in einem Extruder und vorzugsweise einem Zweischneckenextruder herstellbar ist.

14. Zusammensetzung nach Anspruch 13, wobei die Extrusion bei Umgebungstemperatur in der Größenordnung von 20 bis 25 °C oder in der Kälte erfolgt.

15. Zusammensetzung nach Anspruch 1, die in Form einer auf das Gesicht aufzutragenden Maske, die gegebenenfalls Wärme entwickelt, vorliegt und enthält:
- 2 bis 25 Gew.-%, vorzugsweise 5 bis 15 Gew.-% Texturierungsmittel;
- 5 bis 40 Gew.-%, vorzugsweise 10 bis 25 Gew.-% Absorptionsmittel;
- 5 bis 40 Gew.-%, vorzugsweise 15 bis 30 Gew.-% Kaolin;
- 10 bis 70 Gew.-%, vorzugsweise 20 bis 50 Gew.-% Glykol;
- 2 bis 40 Gew.-%, vorzugsweise 5 bis 25 Gew.-% grenzflächenaktiven Stoff; und
- 0 bis 50 Gew.-%, vorzugsweise 15 bis 30 Gew.-% Wasser.

16. Zusammensetzung nach Anspruch 1, die in Form eines pulvrigen, gegebenenfalls anzufeuchtenden Sticks vorliegt und enthält:
- 2 bis 25 Gew.-%, vorzugsweise 5 bis 15 Gew.-% Texturierungsmittel;
- 5 bis 35 Gew.-%, vorzugsweise 10 bis 25 Gew.-% Absorptionsmittel;
- 20 bis 80 Gew.-%, vorzugsweise 30 bis 60 Gew.-% Öl;
- 0 bis 60 Gew.-%, vorzugsweise 10 bis 50 Gew.-% Glykol;
- 2 bis 30 Gew.-%, vorzugsweise 10 bis 20 Gew.-% grenzflächenaktiven Stoff; und
- 0 bis 30 Gew.-%, vorzugsweise 10 bis 20 Gew.-% Wasser.

17. Verwendung einer Zusammensetzung nach einem der vorhergehenden Ansprüche zur kosmetischen Behandlung der Haut des Gesichts und des Körpers, der Haare, der Kopfhaut oder der Schleimhäute.
